# FASCICULE DE BREVET EUROPEEN

(11) **EP 2 959 828 B1**
(45) Date de publication et mention de la délivrance du brevet: **23.11.2016**
(21) Numéro de dépôt: 15171009.2
(22) Date de dépôt: 08.06.2015
(51) Int. Cl.: A61B 5/00, A61N 1/372, A61B 5/07

(54) **ENSEMBLE HYBRIDE FORMANT DISPOSITIF MÉDICAL IMPLANTABLE ACTIF**
HYBRID-EINHEIT, DIE EINE AKTIVE IMPLANTIERBARE MEDIZINISCHE VORRICHTUNG BILDET
HYBRID ASSEMBLY FORMING AN ACTIVE IMPLANTABLE MEDICAL DEVICE

(30) Priorité: 25.06.2014 FR 1455899
(43) Date de publication de la demande: 30.12.2015
(73) Titulaire: Sorin CRM SAS, 92140 Clamart Cedex (FR)
(72) Inventeur: Regnier, Willy, 91160 Longjumeau (FR)
(74) Mandataire: Dupuis-Latour, Dominique

(56) Documents cités:
- EP-A1- 2 719 422
- US-B1- 6 409 674
- US-B1- 7 634 313

## Description

L'invention concerne les "dispositifs médicaux implantables actifs" tels que définis par la directive 90/385/CEE du 20 juin 1990 du Conseil des communautés européennes, et plus précisément la détection des potentiels électriques produits par les organes et/ou la stimulation électrique de ces organes, notamment dans des applications de diagnostic et de thérapie cardiaque.

Toutefois, bien que dans la suite on décrira principalement un ensemble de détection/stimulation cardiaque, cette application n'est pas limitative de l'invention qui, comme on le comprendra, peut être également appliquée, *mutatis mutandis,* à la détection/stimulation d'autres organes tels que système nerveux (notamment pour la stimulation du cerveau ou des nerfs), réseau artériel ou lymphatique, système digestif (estomac, intestin) ou encore le système respiratoire.

Dans le cas du coeur, l'invention vise plus particulièrement la situation des patients en insuffisance cardiaque (HF, *Heart Failure*), auxquels est proposée l'implantation d'un dispositif de resynchronisation cardiaque de type dit CRT-P (stimulateur cardiaque) ou CRT-D (stimulateur avec en outre une fonction de défibrillateur).

La thérapie vise à resynchroniser la contraction des deux ventricules entre eux, et si nécessaire celle des ventricules par rapport à l'oreillette, de manière à améliorer l'état du patient par optimisation des différentes phases du cycle hémodynamique. Pour cela, les dispositifs mettent en oeuvre une technique dite "CRT" (*Cardiac Resynchronisation Therapy*) ou "BVP" (*Bi-Ventricular Pacing*) consistant à délivrer en tant que de besoin des impulsions électriques assurant une stimulation conjointe et permanente des deux ventricules, gauche et droit, afin de resynchroniser les contractions de ces derniers.

En ce qui concerne le dispositif implanté, celui-ci nécessite l'implantation dans le ventricule droit d'une sonde endocavitaire conventionnelle de stimulation et, pour la stimulation du ventricule gauche, d'une sonde introduite dans le réseau veineux coronaire via le sinus coronaire, de manière à placer l'électrode de stimulation de cette sonde contre la paroi du ventricule gauche. Une alternative consiste à utiliser comme sonde ventriculaire gauche une sonde épicardique, introduite dans le sac péricardique et fixée sur la paroi externe du muscle cardiaque. Le dispositif met également souvent en oeuvre une troisième sonde, placée dans la cavité auriculaire droite, permettant de détecter la contraction de l'oreillette afin de synchroniser sur celle-ci la stimulation des ventricules, en respectant la chronologie du délai atrioventriculaire.

Ces sondes, qu'elles soient endocavitaires ou coronaires, sont introduites via le réseau veineux du patient, ce qui peut créer des complications telles que déplacement, rupture d'isolant ou de conducteur, développement de fibroses, etc.

Pour réduite ces risques, une nouvelle génération de dispositifs a été développée, qui se présentent sous forme de capsules autonomes implantables dans une cavité cardiaque (ventricule, oreillette ou même cavité cardiaque gauche artérielle), et sont généralement désignées "capsules *leadless*". Ces capsules sont dépourvues de toute liaison physique à un dispositif principal implanté (tel qu'un boitier de générateur d'impulsions de stimulation) ou non implanté (périphérique externe tel que programmateur ou dispositif de *monitoring* pour le suivi à distance du patient). Elles sont qualifiées pour cette raison de *leadless,* pour les distinguer des électrodes disposées à l'extrémité distale d'une sonde (*lead*) conventionnelle, parcourue sur toute sa longueur par un ou plusieurs conducteurs reliant par voie galvanique l'électrode distale à un connecteur situé à l'extrémité opposée, proximale, de la sonde, ce connecteur étant destiné à être branché sur le boitier du générateur d'impulsions.

Ces capsules *leadless* peuvent avantageusement remplacer les sondes endocavitaires conventionnelles telles que les sondes ventriculaires droites et auriculaires droites, ou encore les sondes épicardiques, mais compte tenu de leur taille elles ne peuvent pas se substituer aux sondes de stimulation du ventricule gauche introduites dans le réseau coronaire veineux, sondes qui sont nécessaires pour la détection/stimulation du ventricule gauche, donc pour l'application d'une thérapie CRT. De plus, le réseau artériel endocavitaire (donnant donc accès aux cavités gauches) demeure toujours extrêmement risqué, même avec une capsule *leadless,* du fait des risques graves d'hémorragie ou de formation de caillots, susceptibles de former des emboles artériels.

D'autre part, en ce qui concerne les sondes coronaires gauches, la nécessité d'utiliser pour leur implantation un fil-guide de pose, la norme standard des connecteurs de sonde gauche multipolaires (normes IS-4 ou DF-4), ainsi que la nécessité d'une lumière centrale ménagée dans le corps de sonde pour l'introduction du fil-guide, sont des contraintes qui limitent la possibilité de réduire le diamètre de ces sondes coronaires et donc d'atteindre de nouvelles zones cibles de stimulation du ventricule gauche qui restent difficilement atteignables aujourd'hui.

Le US 7 634 313 B1 décrit un ensemble de stimulation biventriculaire combinant :
- pour la stimulation du ventricule droit, un stimulateur conventionnel avec un boitier de générateur connecté à une sonde endocavitaire également conventionnelle, et
- pour la stimulation du ventricule gauche, une capsule *leadless* implantée en épicardique sur la paroi externe du ventricule gauche et communiquant par une liaison sans fil avec le générateur, celui-ci jouant un rôle du maître et la capsule *leadless* celui du satellite.

Avec cet ensemble subsistent toutefois les problèmes évoqués ci-dessus, ainsi que ceux propres aux stimulateurs conventionnels (volume du générateur, difficulté à atteindre le sinus coronaire avec la sonde, implantation avec fil-guide de pose, etc.) et, enfin, la nécessité d'une intervention par voie transthoracique pour la pose de la capsule épicardique.

Le but de l'invention est de proposer un dispositif permettant de pallier les limitations exposées ci-dessus.

Essentiellement, l'invention propose de constituer un ensemble comprenant, d'une part, un dispositif qui sera par la suite qualifié de "capsule hybride" pour la détection/stimulation du ventricule gauche et, d'autre part, de capsules *leadless* pour la détection/stimulation des cavités droites (ventricule droit et, le cas échéant, oreillette droite).

On entendra par "capsule hybride" un dispositif constitué :
- d'un corps de même forme et de même configuration qu'une capsule *leadless,* avec une architecture électronique basse consommation, une source d'énergie miniaturisée et des moyens de communication sans fil avec d'autres capsules,
- où ce corps est pourvu d'une sonde prolongeant directement le corps de la capsule sans solution de continuité (c'est-à-dire sans connecteur intermédiaire), de manière à former un dispositif monobloc et entièrement autonome,
- cette sonde étant de surcroit de type "microsonde", c'est-à-dire une sonde miniaturisée de très faible diamètre (typiquement au plus 1 French, soit 0,33 mm) et dépourvue de lumière interne, formée d'un câble de coeur revêtu d'une couche d'isolement avec, en région distale, une ou plusieurs zones sélectivement dénudées formant électrodes de détection/stimulation.

Plus précisément, l'ensemble de l'invention comprend, de manière en elle-même connue d'après le US 7 634 313 B1 précité :
- une unité autonome sous-cutanée comprenant : un corps étanche logeant des circuits électroniques et des moyens d'alimentation électrique de ces circuits électroniques ; et prolongeant ce corps, au moins une sonde de détection et/ou de délivrance de thérapie, cette sonde comprenant au moins une électrode de détection et/ou de délivrance de thérapie ; et
- au moins une capsule *leadless* autonome intracorporelle apte à être implantée dans ou contre un organe et comprenant : un corps étanche logeant des circuits électroniques et des moyens d'alimentation électrique de ces circuits ; des moyens d'ancrage à une paroi dudit organe ; et au moins une électrode de détection et/ou de délivrance de thérapie apte à venir en contact direct avec ledit organe.

L'unité autonome et la capsule *leadless* comprennent chacune des moyens émetteurs/récepteurs de communication sans fil mutuelle par voie intracorporelle, l'unité autonome opérant en tant que maitresse et la ou les capsules *leadless* opérant en tant qu'esclaves sous le contrôle de l'unité autonome maitresse.

L'unité autonome comprend en outre des moyens de centralisation des données transmises par les capsules *leadless,* et des moyens de communication avec l'extérieur, aptes à opérer une télétransmission, vers un équipement distant, des données recueillies par les moyens de centralisation de données.

De façon caractéristique de l'invention :
- la au moins une sonde de détection et/ou de délivrance de thérapie est une microsonde comprenant au moins un microcâble formé d'un câble de coeur électriquement conducteur relié à un pôle desdits circuits électroniques, avec une couche d'isolement entourant le câble de coeur et comprenant au moins une zone sélectivement dénudée ménagée dans la couche d'isolement et destinée à former ladite électrode de détection et/ou de délivrance de thérapie, le diamètre de la microsonde étant d'au plus 1 French (0,33 mm) dans sa région la plus distale comprenant ladite zone sélectivement dénudée ; et
- l'unité autonome est une capsule hybride dont le volume est d'au plus 1 cm³ et dans laquelle : la capsule hybride est dépourvue de connecteur électrique entre la microsonde et les circuits électroniques, la au moins une microsonde prolonge ledit corps d'un côté de celui-ci, et le câble de coeur du microcâble ou de chaque microcâble est directement relié à un pôle respectif des circuits électroniques de la capsule hybride.

Selon diverses caractéristiques subsidiaires avantageuses :
- la capsule hybride est dépourvue de moyens d'ancrage à une paroi d'un organe ;
- le corps étanche de la capsule hybride est un corps métallique comprenant au moins une traversée hermétique et électriquement isolante pour le passage de la liaison du câble de coeur du microcâble, ou de chaque câble de coeur de chaque microcâble, au pôle respectif des circuits électroniques contenus dans le corps de la capsule hybride ;
- le corps de la capsule supporte, du côté opposé au côté prolongé par la microsonde, une antenne d'émission/réception pour la communication sans fil avec ledit équipement distant ; et
- la au moins une microsonde de détection et/ou de délivrance de thérapie comprend, dans la région proximale de la microsonde attenante au corps de la capsule hybride, une zone de transition à gradient de raideur variable, décroissant dans le sens distal.

On va maintenant décrire un exemple de mise en oeuvre de la présente invention, en référence aux dessins annexés où les mêmes références désignent d'une figure à l'autre des éléments identiques ou fonctionnellement semblables.
La Figure 1 montre, en situation d'implantation, les différents éléments constituant un ensemble CRT selon l'état de la technique.
La Figure 2 montre, en situation d'implantation, les différents éléments constituant un ensemble CRT selon l'invention.
La Figure 3 est une vue agrandie de la capsule hybride de l'ensemble de la Figure 2.
La Figure 4 est un détail montrant la manière dont est réalisée la liaison électrique des conducteurs de la microsonde aux circuits internes de la capsule hybride.
La Figure 5 est un schéma fonctionnel décrivant les interactions entre les différents éléments de l'ensemble de l'invention.

On va maintenant décrire un exemple de réalisation de l'invention, appliquée à un ensemble de type resynchroniseur cardiaque (CRT).

Comme cela a été expliqué en introduction, cet exemple ne revêt qu'un caractère illustratif, l'invention pouvant être mise en oeuvre dans le cadre de configurations très variées de détection/stimulation, de surcroit dans un contexte qui n'est pas nécessairement lié à un diagnostic et/ou à une thérapie cardiaque.

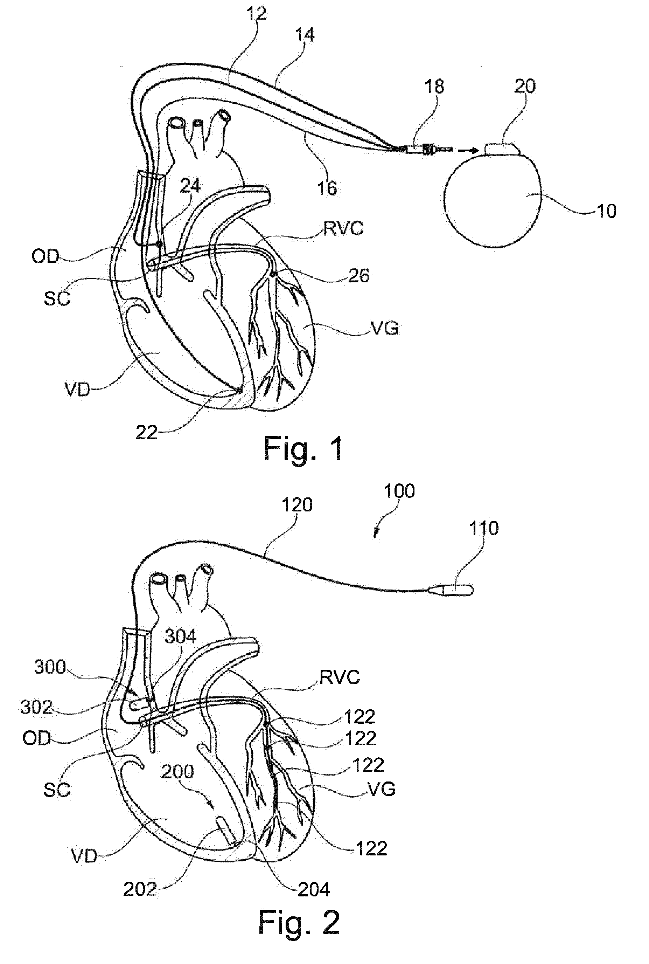

La Figure 1 montre, en situation d'implantation, les différents éléments constitutifs d'un ensemble CRT selon l'état de la technique.

Cet ensemble comporte un générateur 10 de stimulateur CRT, par exemple de la famille *Paradym CRT de* Sorin CRM, Clamart, France. Ce générateur se présente sous forme d'un boitier de volume 30 cm³ environ auquel sont couplées trois sondes 12, 14 et 16 au moyen d'un connecteur 18 enfiché dans une tête de connecteur 20 du générateur 10, typiquement un connecteur de type normalisé IS-4. Le générateur 10 comprend une pile de longue durée pour l'alimentation des circuits internes de commande et de détection/stimulation, dont la puissance moyenne consommée est de l'ordre de 30 µW.

Les sondes de l'ensemble CRT comprennent une sonde ventriculaire droite 12 de type endocavitaire introduite dans le réseau veineux, comprenant un corps de sonde de diamètre typique 4 French (1,33 mm), terminé à son extrémité distale par une tête de sonde portant une électrode de détection/stimulation 22 ancrée au fond de la cavité du ventricule droit VD.

L'ensemble peut également (de manière optionnelle) comprendre une sonde auriculaire droite 14 de type endocavitaire, de structure comparable à celle de la sonde 12 avec un corps de sonde terminé à son extrémité distale par une tête de sonde implantée dans l'oreillette droite OD et pourvue d'une électrode de détection auriculaire 24.

Pour la détection/stimulation du ventricule gauche il n'est pas possible, ou tout au moins extrêmement risqué, d'utiliser une sonde endocavitaire, et pour cette raison on utilise généralement comme sonde ventriculaire gauche 16 une sonde introduite dans le réseau veineux coronaire SVC via le sinus coronaire SC débouchant dans le ventricule droit. Cette sonde coronaire 16 est pourvue à son extrémité distale d'une électrode 26 positionnée en appui contre la paroi du ventricule gauche VG de manière à pouvoir stimuler celui-ci dans la zone de cette électrode. En variante, la sonde ventriculaire gauche 16 peut être une sonde épicardique, introduite entre la paroi du myocarde et le sac épicardique entourant ce dernier, de manière à venir, de la même façon, en contact avec la paroi externe du muscle ventriculaire à stimuler.

La Figure 2 est homologue de la Figure 1, mais pour un ensemble CRT 100 selon l'invention.

Cet ensemble comporte, en lieu et place du générateur 10, un dispositif ci-après dénommé "capsule hybride" 100, associé à une unique sonde 120 couplée au corps 110 de la capsule hybride par un système de liaison simplifié, permanent, remplaçant un connecteur tel que le connecteur IS-4 18 de l'ensemble selon l'état de la technique illustré Figure 1. En d'autres termes, la sonde 120 prolonge le corps 110 de la capsule sans solution de continuité du fait de l'absence de connecteur.

La sonde 120 est une sonde de type "microsonde" telle que décrite notamment dans le EP 2 719 422 A1 (Sorin CRM). Il s'agit d'une sonde de très faible diamètre dans sa partie distale, typiquement un diamètre inférieur à 1,5 French (0,5 mm), de préférence d'au plus 1 French (0,33 mm). Cette sonde est réalisée à partir d'au moins un microcâble constitué lui-même d'un câble de coeur électriquement conducteur revêtu d'une couche d'isolement entourant le câble de coeur et présentant au moins une zone sélectivement dénudée ménagée dans la couche d'isolement pour former une électrode de détection/stimulation. Diverses structures de microcâbles sont notamment décrites dans le EP 2 581 107 A1 (Sorin CRM) auquel on pourra se référer pour de plus amples détails. Avantageusement, comme décrit dans le EP 2 719 422 A1 précité, une pluralité de ces microcâbles sont assemblés ensemble en un toron de microcâbles, chacun électriquement indépendant, de manière à obtenir une microsonde multipolaire dotée de plusieurs électrodes 122 sélectionnables distinctement. Une telle microsonde multipolaire permet notamment de mettre en oeuvre une fonction dite de "repositionnement électrique" consistant à sélectionner, parmi plusieurs points de stimulation correspondant à plusieurs électrodes respectivement reliées à l'un des microcâbles de la sonde, celui assurant la meilleure efficacité. Cette sélection peut être opérée aussi bien au moment de l'implantation de la sonde qu'ultérieurement, en effectuant à intervalles réguliers des tests pour vérifier que le site initialement choisi est toujours optimal, et éventuellement en sélectionner un autre dans le cas contraire.

La partie distale, active, de la microsonde 120 est implantée dans le réseau veineux coronaire RVC de manière que les électrodes 122 soient en contact avec différentes zones de la paroi du ventricule gauche VG. Les diverses électrodes 122 peuvent être constituées par une pluralité de régions dénudées d'une zone monopolaire (ces électrodes étant donc toutes actives et reliées électriquement en parallèle), ou bien par des électrodes différentes, sélectivement commutables, d'une microsonde multipolaire.

L'ensemble de l'invention comporte en outre une capsule *leadless* 200 implantée dans le ventricule droit VD. Cette capsule est de type *leadless,* c'est-à-dire qu'elle est dépourvue de toute liaison physique à un dispositif principal implanté (tel que le générateur 10 de la Figure 1) ou non implanté. Une telle capsule *leadless* comporte un corps 202 muni à l'une de ses extrémités d'un organe d'ancrage 204, généralement une vis hélicoïdale saillante prolongeant axialement le corps 202 de la capsule, et destiné à pénétrer dans le tissu cardiaque par vissage au site d'implantation, de la même manière que pour les sondes à vis conventionnelles.

Le EP 2 394 695 A1 (Sorin CRM) décrit un tel type de capsule *leadless* à vis, ainsi qu'un accessoire permettant son implantation au site choisi par accostage de la vis axiale, entrainement en rotation de la capsule pour fixer celle-ci de façon permanente à la paroi cardiaque où elle se trouvera maintenue par la vis axiale d'ancrage, puis retrait de l'accessoire, la capsule restant alors librement fixée à la paroi cardiaque.

Le corps 202 d'une telle capsule *leadless* se présente habituellement sous une forme générale cylindrique avec une longueur de 20 à 40 mm, un diamètre extérieur inférieur à 6 mm (2 French, dimension imposée par le calibre de la voie d'accès via le réseau veineux périphérique), et un volume d'environ 1 cm³.

La capsule *leadless* intègre une architecture électronique à basse consommation, typiquement 5 à 8 µW, ce qui permet de l'alimenter par un système de récupération d'énergie ou *harvester* (décrit par exemple dans les EP 2 638 930 A1 (Sorin CRM) ou EP 2 639 845 A1 (Sorin CRM)) en lieu et place d'une pile dont la durée de vie est par nature limitée.

Dans la configuration illustrée Figure 2, il est également prévu une seconde capsule *leadless* 300 d'un type comparable à la capsule 200, comprenant un corps 302 et des moyens 304 d'ancrage à la paroi cardiaque, cette paroi étant celle de l'oreillette droite OD de manière à pouvoir recueillir les signaux de dépolarisation auriculaires.

Les capsules 200 et 300 sont des capsules *leadless* conventionnelles, d'un type en elles-mêmes connu, et ne seront pas décrites plus en détail pour cette raison.

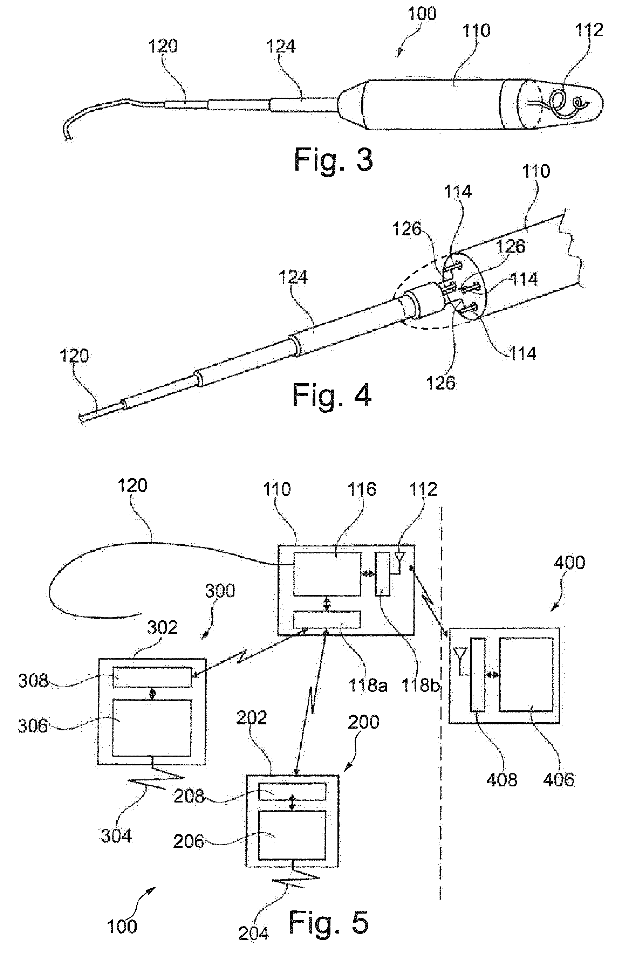

Les Figures 3 et 4 sont des vues agrandies montrant des détails de la capsule hybride 100 de l'ensemble selon l'invention.

La capsule hybride comprend un corps tubulaire métallique 110 (en titane ou alliage) étanche, atraumatique et biocompatible. Le diamètre externe de ce corps est d'au plus 6 mm (18 French), sa longueur est d'au plus 40 mm, et son volume est de l'ordre de 1 cm³. En d'autres termes, le corps de la capsule hybride 110 a sensiblement les mêmes dimensions qu'une capsule *leadless* conventionnelle telle que les capsules 200 et 300.

À l'une des extrémités, le corps 110 de la capsule hybride est pourvu d'une antenne 112 pour une communication sans fil, notamment pour lui permettre de communiquer avec un périphérique externe de type programmateur ou dispositif de télétransmission de données, notamment par télémétrie RF dans la bande MICS (*Medical Implant Communication System*), MEDS, dans les bandes banalisées publiques ISM utilisées par les dispositifs médicaux, ou encore par communication selon les protocoles *Bluetooth.*

Pour la communication HBC entre capsules, il peut être prévu une électrode en forme d'anneau, isolée électriquement du corps 110 de la capsule et de l'antenne 112, destinée à assurer la transmission de données par contact avec les tissus ou le sang via des impulsions électriques dans le corps du patient.

À l'extrémité opposée, la capsule hybride 110 est prolongée par la microsonde 120, avec une région intermédiaire 124 de transition procurant, sur une longueur de l'ordre de 30 mm, un gradient de raideur progressif entre l'extrémité rigide du corps 110 et la partie souple de la microsonde 120.

Comme illustré Figure 4, la liaison entre les microcâbles 126 de la microsonde 120 et les circuits électriques internes contenus dans le corps de la capsule hybride 110 est réalisée par des traversées 114 étanches, qui sont des traversées unipolaires avec une broche conductrice soudée sur une embase solidaire du corps 110 de la capsule hybride et reliées chacune à un microcâble 126 respectif de la microsonde 120.

Comme pour les capsules *leadless* 200 et 300, la capsule hybride 110 intègre une architecture électronique basse consommation, typiquement 5 à 8 µW, alimentée par une batterie ou, en variante, un système *harvester* de récupération d'énergie. Avantageusement, le circuit électronique de la capsule hybride 100 intègre également un ou plusieurs capteurs d'asservissement, par exemple un accéléromètre 3D et une thermistance permettant de mesurer la température corporelle (dans une configuration où le corps 110 de la capsule hybride est implantée en sous-cutané).

La Figure 5 est un schéma fonctionnel illustrant les interactions entre les différents dispositifs de l'ensemble de l'invention.

Chacune des capsules hybride 100 ou *leadless* 200 et 300 comporte des circuits électroniques de commande, respectivement 116, 206, 306, couplés à des moyens émetteurs/récepteurs de communication sans fil, respectivement 108a-118b, 208 et 308, pour permettre la communication mutuelle entre les différentes capsules 100, 200, 300 ainsi que pour la communication de la capsule hybride 110 avec un équipement distant 400. L'équipement distant 400 comprend de son côté des circuits 406 couplés à des moyens émetteurs/récepteurs 408. Cet équipement externe 400 peut être notamment le programmateur d'un praticien, la communication servant alors à interroger l'ensemble implanté, y lire des données stockées en mémoire, en modifier certains paramètres, etc. L'équipement externe 400 peut être également un dispositif de *home monitoring,* c'est-à-dire un dispositif externe de suivi à domicile de l'état du patient, avec éventuellement possibilité de transmission des informations à un site distant, hospitalier ou autre. Le *Smartview Remote Monitoring System* de Sorin CRM est un exemple d'un tel dispositif externe.

La communication entre la capsule hybride 100 et les capsules *leadless* 200 et 300 (via les circuits respectifs 118a, 208 et 308) est une communication intracorporelle de type HBC (*Human Body Communication,* communication par voie intracorporelle), mettant par exemple en oeuvre une technique de communication au moyen d'impulsions transmises via les tissus interstitiels du corps du patient, ces impulsions étant générées, émises, recueillies et détectées par des circuits appropriés par exemple tels que ceux décrits dans les EP 2 441 491 A1 (Sorin CRM) et EP 2 486 953 A1 (Sorin CRM). La communication entre la capsule hybride 110 et le dispositif externe 400 est une communication de télémétrie RF par exemple dans les bandes MICS, MEDS, ISM ou encore utilisant le protocole *Bluetooth.*

La capsule hybride 100 joue un rôle de dispositif maitre, ou concentrateur, dans une architecture de réseau sans fil en étoile, dont les capsules *leadless* 200 et 300 sont des dispositifs esclaves.

Plus précisément, le rôle de la capsule hybride 100 (maitresse) est de :
- détecter l'activité sur le ventricule gauche, recueillie sur les électrodes de la microsonde 120 et transmise directement, par voie galvanique, via les microcâbles 126 de cette sonde ;
- stimuler le ventricule gauche, par envoi d'impulsions appropriées sur les électrodes de la microsonde 120 ;
- recevoir des informations en provenance de la capsule ventriculaire droite 200 et de la capsule auriculaire gauche 300 ;
- envoyer des ordres à ces capsules ventriculaires droite 200 et auriculaire gauche 300 ;
- interagir avec un dispositif 400 extérieur au corps du patient.

Le rôle (esclave) de la capsule *leadless* ventriculaire droite 200 est de :
- détecter l'activité sur le ventricule droit à partir des signaux détectés par l'électrode en contact avec le myocarde dans cette zone, par exemple par la vis d'ancrage 204 si celle-ci joue également le rôle d'électrode ;
- envoyer des informations correspondantes vers la capsule hybride 100 située dans une région sous-cutanée ;
- exécuter les ordres envoyés par la capsule hybride 100, notamment pour la délivrance d'impulsions de stimulation du ventricule droit.

Le rôle (esclave) de la capsule *leadless* auriculaire 300 est de :
- détecter l'activité auriculaire à partir des signaux détectés par l'électrode en contact avec le myocarde dans cette zone, par exemple par la vis d'ancrage 304 si celle-ci joue également le rôle d'électrode ;
- envoyer des informations correspondantes vers la capsule hybride 100 située dans une région sous-cutanée ;
- exécuter les ordres envoyés par la capsule hybride 100, notamment pour la délivrance d'impulsions de stimulation du ventricule droit.

L'invention que l'on vient de décrire présente un certain nombre d'avantages, parmi lesquels on peut citer :
- par rapport à un générateur de stimulateur conventionnel, la capsule hybride 100 selon l'invention permet une miniaturisation poussée et une réduction importante de la consommation d'énergie : dans l'invention, une capsule avec un corps 110 de 1 cm³ environ, associée à une microsonde de 1 French (0,33 mm), remplace avantageusement un générateur de 30 cm³ sur lequel est branchée une sonde coronaire de calibre 4 French (1,33 mm) ;
- simplification du système grâce à la suppression complète du connecteur ;
- possibilité d'utiliser pour la pose un microcathéter tel que celui décrit dans le EP 2 682 151 A1 (Sorin CRM), qui permet d'implanter une microsonde en conservant les accessoires de pose traditionnels (introducteur, fil-guide, cathéter, etc.), qui seront utilisés de la même façon qu'antérieurement, donc avec le même geste chirurgical ;
- gain de temps à l'implantation, du fait de la suppression de l'étape de connexion de la sonde sur la tête de connecteur du stimulateur, ainsi que de l'étape de test de la sonde pour vérifier la conformité de l'interaction sonde/générateur ;
- système par conception compatible avec les examens par imagerie par résonance magnétique (IRM/MRI), qui ne risquent pas de détruire les électrodes du système grâce à l'utilisation de pièces biocompatibles en matière plastique ou silicone et de pièces rigides en titane
- fiabilité accrue grâce à une conception simplifiée et une réduction du nombre de pièces ;
- possibilité de réduire encore le diamètre de la capsule hybride en deçà de 20 French (6,6 mm), en fonction du volume de l'électronique et du système d'alimentation en énergie.

De nombreuses variantes de l'invention peuvent être envisagées, notamment dans des configurations où l'ensemble est adapté à d'autres applications qu'une thérapie CRT, ou même à une autre application qu'une thérapie cardiaque. En particulier :
- la microsonde peut être une microsonde apte à être introduite dans une veine, dans une artère, dans le système lymphatique, dans le système nerveux (notamment pour une thérapie de type VNS (*Vagus Nerve Stimulation,* stimulation du nerf vague), et de façon générale dans toute cavité corporelle ;
- la capsule hybride peut comporter plusieurs sondes qui prolongent le corps 110 de la capsule proprement dite ;
- les capsules *leadless* de l'ensemble selon l'invention peuvent être adaptées à la détection ou à la délivrance de thérapies non seulement au coeur, mais également à d'autres organes tels que l'estomac, l'intestin, le poumon, le cerveau, etc.
- l'ensemble peut comprendre une pluralité de capsules hybrides, dont l'une est maitresse et dont l'(es)autre(s) est(sont) esclave(s). Ces capsules hybrides additionnelles joueront alors, fonctionnellement, le même rôle que les capsules *leadless,* à la différence près que la détection/stimulation sera opérée par la région active, distale de la microsonde de la capsule hybride additionnelle, au lieu de l'être directement par la vis d'ancrage ou l'électrode au point de contact avec la paroi d'une capsule *leadless.*

## Revendications

1. Un ensemble formant dispositif médical implantable actif, comprenant :
- une unité autonome (100) sous-cutanée comprenant :
• un corps étanche (110) logeant des circuits électroniques et des moyens d'alimentation électrique de ces circuits électroniques ; et
• prolongeant ce corps, au moins une sonde (120) de détection et/ou de délivrance de thérapie, cette sonde comprenant au moins une électrode (122) de détection et/ou de délivrance de thérapie ; et
- au moins une capsule *leadless* autonome (200 ; 300) intracorporelle apte à être implantée dans ou contre un organe et comprenant :
• un corps étanche (202 ; 302) logeant des circuits électroniques et des moyens d'alimentation électrique de ces circuits ;
• des moyens d'ancrage (204 ; 304) à une paroi dudit organe ; et
• au moins une électrode (204 ; 304) de détection et/ou de délivrance de thérapie apte à venir en contact direct avec ledit organe,
dans lequel l'unité autonome (100) et la capsule *leadless* (200 ; 300) comprennent chacune des moyens émetteurs/récepteurs (118a; 208; 308) de communication sans fil mutuelle par voie intracorporelle, l'unité autonome opérant en tant que maitresse et la ou les capsules *leadless* opérant en tant qu'esclaves sous le contrôle de l'unité autonome maitresse,
et dans lequel l'unité autonome (100) comprend en outre :
des moyens de centralisation des données transmises par les capsules *leadless,* et
des moyens (118b, 112) de communication avec l'extérieur, aptes à opérer une télétransmission, vers un équipement distant (400), des données recueillies par les moyens de centralisation de données,
ensemble **caractérisé en ce que** :
- ladite au moins une sonde de détection et/ou de délivrance de thérapie est une microsonde (120) comprenant au moins un microcâble (126) formé d'un câble de coeur électriquement conducteur relié à un pôle desdits circuits électroniques, avec une couche d'isolement entourant le câble de coeur et comprenant au moins une zone sélectivement dénudée ménagée dans la couche d'isolement et destinée à former ladite électrode (122) de détection et/ou de délivrance de thérapie, le diamètre de la microsonde étant d'au plus 1 French (0,33 mm) dans sa région la plus distale comprenant ladite zone sélectivement dénudée ; et
- ladite unité autonome est une capsule hybride dont le volume est d'au plus 1 cm³ et dans laquelle :
• la capsule hybride est dépourvue de connecteur électrique entre la microsonde et les circuits électroniques,
• la au moins une microsonde (120) prolonge ledit corps (110) d'un côté de celui-ci, et
• le câble de coeur du microcâble (126), ou de chaque microcâble, est directement relié à un pôle respectif des circuits électroniques de la capsule hybride.

2. L'ensemble de la revendication 1, dans lequel la capsule hybride est dépourvue de moyens d'ancrage à une paroi d'un organe.

3. L'ensemble de la revendication 1, dans lequel le corps étanche (110) de la capsule hybride est un corps métallique comprenant au moins une traversée hermétique et électriquement isolante (114) pour le passage de la liaison du câble de coeur du microcâble (126), ou de chaque câble de coeur de chaque microcâble, au pôle respectif des circuits électroniques contenus dans le corps (110) de la capsule hybride.

4. L'ensemble de la revendication 1, dans lequel le corps de la capsule supporte, du côté opposé au côté prolongé par la microsonde, une antenne (112) d'émission/réception pour la communication sans fil avec ledit équipement distant.

5. L'ensemble de la revendication 1, dans lequel la au moins une microsonde (120) de détection et/ou de délivrance de thérapie comprend, dans la région proximale de la microsonde attenante au corps (110) de la capsule hybride, une zone de transition (124) à gradient de raideur variable, décroissant dans le sens distal.

## Patentansprüche

1. Anordnung, die eine aktive implantierbare medizinische Vorrichtung bildet und Folgendes umfasst:
- eine subkutane autonome Einheit (100), die Folgendes umfasst:
• einen dichten Körper (110), der elektronische Schaltungen und Mittel für die elektrische Versorgung dieser elektronischen Schaltungen aufnimmt; und
• diesen Körper verlängernd wenigstens eine Sonde (120) für die Detektion und/oder Verabreichung einer Therapie, wobei diese Sonde wenigstens eine Elektrode (122) für die Detektion und/oder Verabreichung einer Therapie aufweist; und
- wenigstens eine intrakorporale, autonome leitungslose Kapsel (200; 300), die in oder an einem Organ implantiert werden kann und Folgendes umfasst:
• einen dichten Körper (202; 302), der elektronische Schaltungen und Mittel für die elektrische Versorgung dieser Schaltungen aufnimmt;
• Mittel (204; 304) für die Verankerung an einer Wand des Organs; und
• wenigstens eine Elektrode (204; 304) für die Detektion und/oder Verabreichung einer Therapie, die mit dem Organ in direkten Kontakt gelangen kann,
wobei die autonome Einheit (100) und die leitungslose Kapsel (200; 300) jeweils Sender/Empfänger-Mittel (118a; 208; 308) für die gegenseitige drahtlose Kommunikation über einen intrakorporalen Weg umfassen, wobei die autonome Einheit als Master arbeitet und wobei die eine oder die mehreren leitungslosen Kapseln als Slaves unter der Steuerung der autonomen Master-Einheit arbeiten,
und wobei die autonome Einheit (100) außerdem Folgendes umfasst:
• Zentralisierungsmittel für Daten, die von den leitungslosen Kapseln gesendet werden, und
• Mittel (118b, 112) für die Kommunikation mit der äußeren Umgebung, die eine Teleübertragung von Daten, die von den Datenzentralisierungsmitteln gesammelt werden, zu einem entfernten Gerät (400) ausführen können, wobei die Anordnung **dadurch gekennzeichnet ist, dass**:
- die wenigstens eine Detektions- und/oder Therapieverabreichungssonde eine Mikrosonde (120) ist, die wenigstens ein Mikrokabel (126) enthält, das aus einem elektrisch leitenden Kernkabel, das mit einem Pol der elektronischen Schaltungen verbunden ist, und einer das Kernkabel umgebenden Isolierschicht gebildet ist und wenigstens eine wahlweise abisolierte Zone aufweist, die in der Isolierschicht ausgebildet ist und dazu bestimmt ist, die Elektrode (122) für die Detektion und/oder Therapieverabreichung zu bilden, wobei der Durchmesser der Mikrosonde in ihrem am weitesten distalen Bereich, der die wahlweise abisolierte Zone aufweist, höchstens 1 French (0,33 mm) beträgt; und
- die autonome Einheit eine Hybridkapsel ist, deren Volumen höchstens 1 cm³ beträgt und wobei:
• die Hybridkapsel keinen elektrischen Verbinder zwischen der Mikrosonde und den elektronischen Schaltungen aufweist,
• die wenigstens eine Mikrosonde (120) den Körper (110) auf einer seiner Seiten verlängert und
• das Kernkabel des Mikrokabels (126) oder jedes Mikrokabels direkt mit einem entsprechenden Pol der elektronischen Schaltungen der Hybridkapsel verbunden ist.

2. Anordnung nach Anspruch 1, wobei die Hybridkapsel keine Mittel für die Verankerung an einer Wand eines Organs aufweist.

3. Anordnung nach Anspruch 1, wobei der dichte Körper (110) der Hybridkapsel ein metallischer Körper ist, der wenigstens einen hermetisch dichten und elektrisch isolierenden Durchlass (114) für den Durchgang der Verbindung des Kernkabels des Mikrokabels (126) oder jedes Kernkabels jedes Mikrokabels mit dem entsprechenden Pol der elektronischen Schaltungen, die in dem Körper (110) der Hybridkapsel enthalten sind, umfasst.

4. Anordnung nach Anspruch 1, wobei der Körper der Kapsel auf der Seite, die der durch die Mikrosonde verlängerten Seite gegenüberliegt, eine Sende-/Empfangsantenne (112) für die drahtlose Kommunikation mit dem entfernten Gerät trägt.

5. Anordnung nach Anspruch 1, wobei die wenigstens eine Mikrosonde (120) für die Detektion und/oder Therapieverabreichung in dem proximalen Bereich der Mikrosonde, der an den Körper (110) der Hybridkapsel angrenzt, eine Übergangszone (124) mit einem variablen Radiusgradienten, der in distaler Richtung abnimmt, umfasst.

## Claims

1. An active implantable medical device assembly, comprising:
- a sub-cutaneous autonomous unit (100) comprising:
. a tight body (110) housing electronic circuits and electric supply means of these electronic circuits; and
. extending this body, at least one detection and/or therapy delivery lead (120), this lead comprising at least one detection and/or therapy delivery electrode (122); and
- at least one intracorporeal autonomous leadless capsule (200; 300) adapted to be implanted in or against an organ and comprising:
. a tight body (202; 302) housing electronic circuits and electric supply means of these electronic circuits;
. means (204; 304) for the anchoring to a wall of said organ; and
. at least one detection and/or therapy delivery electrode (204; 304) adapted to come into direct contact with said organ,
wherein the autonomous unit (100) and the leadless capsule (200; 300) each comprise emitter/receiver means (118a; 208; 308) for mutual intracorporeal wireless communication, the autonomous unit operating as a master and the leadless capsule(s) operating as slaves under the control of the master autonomous unit,
and wherein the autonomous unit (100) further comprises:
. means for centralizing the data transmitted by the leadless capsules, and
. means (118b, 112) for communication with the outside, adapted to operate a remote-transmission, towards a remote equipment (400), of the data collected by the data centralization means,
the assembly being **characterized in that**:
- said at least one detection and/or therapy delivery lead is a microlead (120) comprising at least one microcable (126) formed of an electrically conductive core cable connected to a pole of said electronic circuits, with an insulation layer surrounding the core cable and comprising at least one selectively-naked zone formed in the insulation layer and intended to form said detection and/or therapy delivery electrode (122), the diameter of the microlead being of at most 1 French (0.33 mm) in its most distal region comprising said selectively-naked zone; and
- said autonomous unit is a hybrid capsule whose volume is of at most 1 cm³ and in which:
. the hybrid capsule is devoid of electric connector between the microlead and the electronic circuits,
. the at least one microlead (120) extends said body (110) on one side of the latter, and
. the core cable of the microcable (126), or of each microcable, is directly connected to a respective pole of the electric circuits of the hybrid capsule.

2. The assembly of claim 1, wherein the hybrid capsule is devoid of means for the anchoring to a wall of an organ.

3. The assembly of claim 1, wherein the tight body (110) of the hybrid capsule is a metal body comprising at least one hermetic and electrically insulating feed-through (114) for the passage of the connection of the core cable of the microcable (126), or of each core cable of each microcable, to the respective pole of the electronic circuits contained in the body (110) of the hybrid capsule.

4. The assembly of claim 1, wherein the body of the capsule supports, on the side opposite to the side extended by the microlead, an emitter/receiver antenna (112) for wireless communication with said remote equipment.

5. The assembly of claim 1, wherein the at least one detection and/or therapy delivery microlead (120) comprises, in the proximal region of the microlead adjacent to the body (110) of the hybrid capsule, a zone of transition (124) with a variable stiffness gradient, decreasing in the distal direction.
